# EUROPÄISCHE PATENTSCHRIFT

(11) **EP 3 412 816 B1**
(45) Veröffentlichungstag und Bekanntmachung des Hinweises auf die Patenterteilung: **14.08.2019**
(21) Anmeldenummer: 18167028.2
(22) Anmeldetag: 12.04.2018
(51) Int. Cl.: D04B 1/26, D04B 1/18, D04B 1/10, A61F 13/08

(54) **KOMPRESSIONSGESTRICK AUS EINEM GRUNDGESTRICK UND EINEM DARIN EINGELEGTEN ELASTISCHEN SCHUSSFADEN, DARAUS HERGESTELLTER KOMPRESSIONSARTIKEL SOWIE VERFAHREN ZUR HERSTELLUNG EINES KOMPRESSIONSGESTRICKS**
COMPRESSION KNITTED FABRIC FROM A BASE KNITTED FABRIC AND AN INSERTED ELASTIC WEFT THREAD, COMPRESSION ARTICLE MADE THEREWITH AND METHOD FOR PRODUCING A COMPRESSION KNITTED FABRIC
TRICOT DE COMPRESSION D'UN TRICOT DE BASE ET D'UN FILS DE TRAME ÉLASTIQUE INTRODUIT DANS LEDIT TRICOT DE BASE, ARTICLE DE CONTENTION INCORPORANT LEDIT TRICOT DE COMPRESSION AINSI QUE PROCÉDÉ DE FABRICATION D'UN TRICOT DE COMPRESSION

(30) Priorität: 30.05.2017 DE 202017103246 U
(43) Veröffentlichungstag der Anmeldung: 12.12.2018
(73) Patentinhaber: Julius Zorn GmbH, 86551 Aichach (DE)
(72) Erfinder: Buchschuster, Martin, 86405 Meitingen (DE)
(74) Vertreter: Charrier Rapp & Liebau

(56) Entgegenhaltungen:
- EP-A1- 1 895 036
- DE-A1- 2 047 566
- US-A- 3 287 938
- US-A- 3 975 929

## Beschreibung

Die Erfindung betrifft ein Kompressionsgestrick nach dem Oberbegriff des Anspruchs 1 sowie ein Verfahren zur Herstellung eines Kompressionsgestricks nach dem Oberbegriff des Anspruchs 14.

Kompressionsgestricke werden beispielsweise zur Herstellung von Kompressionsartikeln wie Kompressionsstrümpfen oder Kompressionsbandagen verwendet. Solche Kompressionsgestricke können insbesondere als Rundgestricke auf einer Rundstrickmaschine oder neuerdings auch auf Flachstrickmaschinen hergestellt werden. Bei den aus dem Stand der Technik bekannten Kompressionsgestricken ist das Grundgestrick aus wenigstens einem elastischen oder un- elastischen Strickfaden gebildet und in das Grundgestrick ist in jeder zweiten gestrickten Reihe ein kompressionsgebender elastischer Schussfaden eingelegt. Bei dem elastischen Schussfaden kann es sich beispielsweise um einen Elastan- oder Gummifaden oder um ein Umwindegarn mit einem elastischen Kernfaden, um den ein Garn gewickelt ist, handeln.

Aufgrund der Elastizität des Schussfadens erhält das als Rundgestrick ausgebildete, schlauchförmige Kompressionsgestrick in Umfangsrichtung seine kompressiven Eigenschaften. Wenn das schlauchförmige Kompressionsgestrick beispielsweise als Kompressionsstrumpf an einem Bein eines Patienten angelegt wird, erzeugt das Kompressionsgestrick durch die Elastizität des elastischen Schussfadens einen Kompressionsdruck auf das Bein. Andere Kompressionsartikel, wie z.B. Kompressionsärmel, sind ebenfalls aus dem Stand der Technik zur Behandlung von Venenerkrankungen und Lymphinsuffizienzen bekannt.

Wenn als elastischer Schussfaden ein Elastan- oder Gummifaden verwendet wird und dieser, wie bei den bekannten Kompressionsgestricken üblich, als Fang und Flottung zwischen zwei aufeinanderfolgende Maschenreihen des Grundgestricks eingelegt wird, kommt der elastische Schussfaden an den Stellen, an denen er flott liegt, an der Oberfläche des Kompressionsgestricks zum Vorschein. Beim Anlegen des Kompressionsgestricks an einem Körperglied kommt der elastische Schussfaden dadurch mit der Haut des Patienten in Kontakt. Dies kann bei Unverträglichkeiten des Patienten gegenüber dem Material des Schussfadens zu Hautirritationen führen. Bei Verwendung eines Umwindegarns als elastischer Schussfaden weist dieser im Vergleich zu dem Strickfaden des Grundgestricks eine wesentlich größere Dicke auf und das Garn, das bei dem Umwindegarn um den elastischen Kernfaden gewickelt ist, weist eine hohe Reibung auf. Durch die Dicke des elastischen Schussfadens aus einem Umwindegarn und die hohe Reibung der Oberfläche des Umwindegarns ergeben sich Schwierigkeiten beim Anziehen eines aus dem Kompressionsgestrick hergestellten Kompressionsartikels, weil es zu einer starken Reibung der Oberfläche des elastischen Schussfadens an der Haut des Patienten kommt.

Zur Lösung dieses Problems wird in der DE 10 2015 110 313 A1 ein KompressionsRundgestrick vorgeschlagen, das aus wenigstens zwei unterschiedlichen Gestrickbereichen besteht, wobei ein erster Gestrickbereich als kompressiver Gestrickbereich in Form eines herkömmlichen Kompressionsgestricks aus einem elastischen Grundgestrickfaden und einem darin als Fang und Flottung eingelegten Schussfaden ausgeführt ist und ein zweiter Gestrickbereich vorgesehen ist, in dem der elastische Grundgestrickfaden und der elastische, kompressionsgebende Faden miteinander maschenbildend verstrickt sind. Mit dem zweiten Gestrickbereich kann dabei einerseits eine stützende bzw. kompressive Wirkung erzielt werden, wobei die dabei erreichbare Kompression von dem maschenbildend mit dem Grundgestrickfaden verstricktem elastischem Faden bestimmt wird und in Umfangsrichtung weniger hoch ist als bei dem herkömmlichen Kompressionsgestrick des ersten Gestrickbereichs. Allerdings liegt der kompressionsgebende elastische Faden in dem zweiten Gestrickbereich zumindest zu einem großen Teil im Gestrickinneren, da der kompressionsgebende elastische Faden über Maschen im Grundgestrick gebunden ist. Dadurch wird zumindest in dem zweiten Gestrickbereich ein verminderter Kontakt des kompressionsgebenden, elastischen Fadens mit der Haut eines Patienten gewährleistet, wodurch einerseits Hautirritationen vermindert und andererseits das Anziehen des Kompressionsartikels erleichtert wird.

Allerdings führt das Kompressionsrundgestrick, welches in der DE 10 2015 110 313 A1 vorgeschlagen wird, nur zu einer bereichsweisen Verminderung des Kontakts zwischen dem kompressionsgebenden elastischen Faden und der Haut eines Patienten und ermöglicht deshalb auch nur zum Teil eine Erleichterung beim Anziehen eines aus dem Kompressionsgestrick hergestellten Kompressionsartikels.

Hiervon ausgehend liegt der Erfindung die Aufgabe zugrunde, ein gattungsgemäßes Kompressionsgestrick so weiterzubilden, dass eine wesentliche Erleichterung beim Anziehen eines aus dem Kompressionsgestrick hergestellten Kompressionsartikels erzielt und die Gefahr von Hautirritationen weitgehend vermieden werden kann. Eine weitere Aufgabe besteht darin, ein Verfahren zur Herstellung eines solchen Kompressionsgestricks aufzuzeigen, welches insbesondere auf Rundstrickmaschinen oder auch auf Flachstrickmaschinen automatisiert durchführbar ist.
Diese Aufgaben werden mit einem Kompressionsgestrick mit den Merkmalen des Anspruchs 1 sowie mit einem Verfahren zur Herstellung eines Kompressionsgestricks mit den Merkmalen des Anspruchs 14 gelöst. Bevorzugte Ausführungsformen des Kompressionsgestricks und des Verfahrens sind den abhängigen Ansprüchen zu entnehmen.

Das erfindungsgemäße Kompressionsgestrick besteht aus einem Grundgestrick aus wenigstens einem Strickfaden sowie einem in das Grundgestrick eingelegten elastischen Schussfaden, wobei der elastische Schussfaden jeweils in Schussfadenreihen zwischen zwei aufeinanderfolgenden Maschenreihen des Grundgestricks eingelegt ist. Dabei ist der Strickfaden in jeder Maschenreihe des Grundgestricks abwechselnd als Masche und Fang gestrickt, so dass der Strickfaden zwischen zwei Maschen einer Maschenreihe des Grundgestricks jeweils einen Fanghenkel bildet. Der Schussfaden ist zwischen benachbarten Maschenreihen des Grundgestricks jeweils abwechselnd als Fang und Flottung eingelegt, so dass der Schussfaden zwischen zwei Fanghenkel einer Schussfadenreihe flott liegt. Die abwechselnde Reihenfolge von Masche und Fang in aufeinanderfolgenden Maschenreihen des Grundgestricks ist dabei jeweils um eine Masche versetzt angeordnet und die abwechselnde Reihenfolge von Fang und Flottung in aufeinanderfolgenden Schussfadenreihen ist ebenfalls jeweils um eine Masche versetzt angeordnet.

Diese Ausbildung des Grundgestricks und die Einlegung des elastischen Schussfadens zwischen zwei aufeinanderfolgende Maschenreihen des Grundgestricks führt dazu, dass die Fanghenkel des elastischen Schussfadens in der darauf folgenden Masche (also in einer Masche der darauf folgenden Maschenreihe des Grundgestricks) abgeworfen wird und somit an dieser Stelle flott liegt. Insgesamt liegt der elastische Schussfaden damit in jeder Schussfadenreihe frei und ohne Bindungspunkte in dem Grundgestrick. Des Weiteren liegt der Schussfaden in dem Kompressionsgestrick vollständig, d.h. über seine gesamte Länge in Umfangsrichtung eines schlauchförmigen Kompressions-Rundgestricks, innen, d.h. innerhalb des Grundgestricks, und erscheint daher nicht an der Oberfläche des Kompressionsgestricks. Dadurch wird einerseits ein Kontakt zwischen der Oberfläche des elastischen Schussfadens und der Haut eines Patienten (fast) vollständig vermieden, weshalb es kaum zu Hautirritationen kommen kann. Des Weiteren wird durch die vollständige Einbettung des elastischen Schussfadens in dem Grundgestrick das Anziehen eines aus dem Kompressionsgestrick hergestellten Kompressionsartikels erleichtert, weil die raue Oberfläche des beispielsweise als Umwindegarn ausgebildeten Schussfadens kaum in Kontakt mit der Haut eines Patienten kommen kann. Die beim Anziehen eines Kompressionsartikels entstehende Reibung zwischen der Gestrickinnenseite des Kompressionsartikels und der Haut eines Patienten wird bei dem erfindungsgemäßen Kompressionsgestrick im Wesentlichen durch die Reibungseigenschaften der Oberfläche des Strickfadens beeinflusst. Da der Strickfaden im Vergleich zum elastischen Schussfaden in der Regel wesentlich dünner ist und im Vergleich zu einem als Umwindegarn ausgebildeten Schussfaden eine wesentlich glattere Oberfläche aufweist, ist die beim erfindungsgemäßen Gestrick durch den Strickfaden hervorgerufene Reibung auf der Haut des Patienten wesentlich geringer als die bei herkömmlichen Kompressionsgestricken vom dicken und an der Gestrickinnenseite freiliegenden Schussfaden erzeugte Reibung. Daher ermöglicht ein aus dem erfindungsgemäßen Kompressionsgestrick hergestellter Kompressionsartikel ein wesentlich leichteres Anziehen aufgrund der geringen Reibung zwischen der Gestrickinnenseite und der Haut eines Patienten.

Bei dem Strickfaden, aus dem das Grundgestrick beispielsweise in Form eines Rechts-Links-Gestricks gestrickt werden kann, handelt es sich zweckmäßig um einen Faden mit einer begrenzten Elastizität. Zweckmäßig ist die Dehnbarkeit des Strickfadens geringer als die Dehnbarkeit des elastischen Schussfadens. Es ist auch möglich, einen unelastischen Strickfaden zu verwenden.

Für die Anwendung des erfindungsgemäßen Kompressionsgestricks als Kompressionsartikel zur Behandlung von Venenerkrankungen oder von Lymphinsuffizienzen wird bevorzugt als Strickfaden ein Umwindegarn mit einem elastischen Kernfaden eingesetzt. Neben dem Strickfaden und dem in das Grundgestrick eingelegten elastischen Schussfaden können ggf. noch weitere Fäden in das Grundgestrick eingebunden werden, wie z.B. einen maschenbildend eingestrickten gummielastischen Faden, der das Kompressionsgestrick auch in Längsrichtung dehnbar macht.

Bei dem elastischen Schussfaden handelt es sich bevorzugt um ein Umwindegarn mit einem hochelastischen Kernfaden und einem umwickelten Garn. Alternativ kann als elastischer Schussfaden beispielsweise auch ein (unumwundener) Elastan- oder Gummifaden verwendet werden. Der Schussfaden weist bevorzugt eine Dicke im Bereich von 200 dtex bis 1500 dtex auf. Die Dicke des Strickfadens ist demgegenüber meist wesentlich geringer, bspw. um einen Faktor 2 bis 20, liegt jedoch bevorzugt bei mindestens 44 dtex.

Das Grundgestrick kann beispielsweise als Rechts-Links-Gestrick ausgebildet sein. Andere Strickbindungen sind jedoch denkbar.

Zweckmäßig ist das erfindungsgemäße Kompressionsgestrick als Rundgestrick, d.h. in Form eines Schlauchs ausgebildet, so dass aus dem Kompressionsgestrick nahtlose Kompressionsartikel, wie z.B. Kompressionsstrümpfe oder -ärmel, gefertigt werden können.

Das erfindungsgemäße Kompressionsgestrick kann mit dem erfindungsgemäßen Verfahren als Rundgestrick auf einer Rundstrickmaschine oder einer Flachstrickmaschine oder auch als Flachgestrick auf einer Flachstrickmaschine hergestellt werden.

Diese und weitere Vorteile sowie Merkmale des erfindungsgemäßen Kompressionsgestricks ergeben sich aus dem nachfolgend unter Bezugnahme auf die begleitenden Zeichnungen näher beschriebenen Ausführungsbeispiel. Die Zeichnungen zeigen:
- **Figur 1:**: Ein Maschenbild einer Ausführungsform des erfindungsgemäßen Kompressionsgestricks (rechte Warenseite);
- **Figur 1a:**: Ein vergrößerter Ausschnitt des Maschenbilds von Figur 1 (rechte Warenseite);
- **Figur 2:**: Schematische Darstellung des Fadenlaufs der Ausführungsform eines erfindungsgemäßen Kompressionsgestricks gemäß Figur 1;
- **Figur 3:**: Technische Patrone des Kompressionsgestricks von Figur 1.

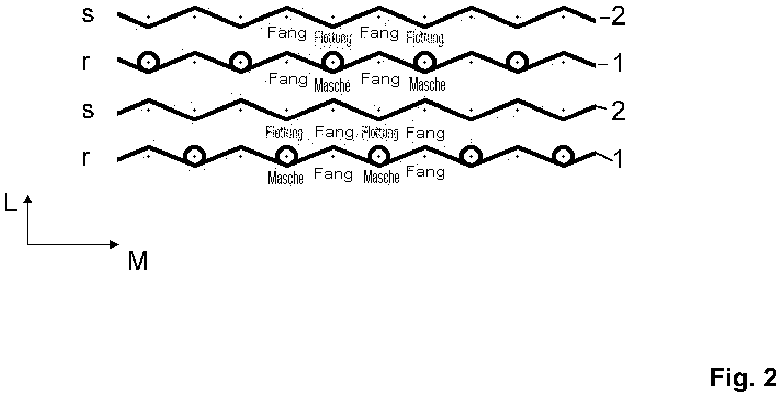

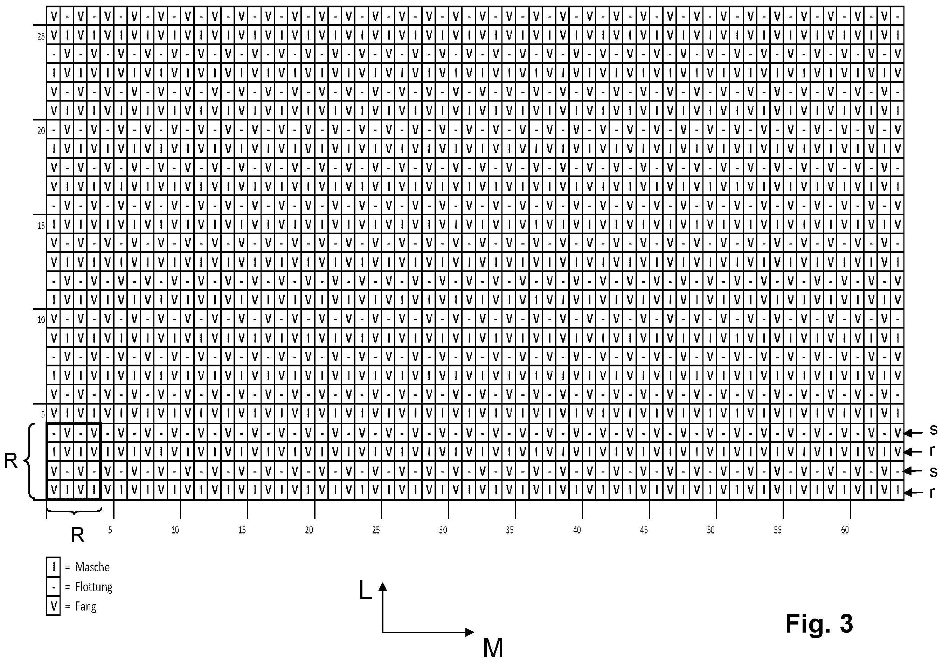

In der Zeichnung des Fadenlaufs der **Figur 2** und der technischen Patrone der **Figur 3** sind folgende Symbole mit folgender Bedeutung verwendet:
I = Masche
- = Flottung
V = Fang

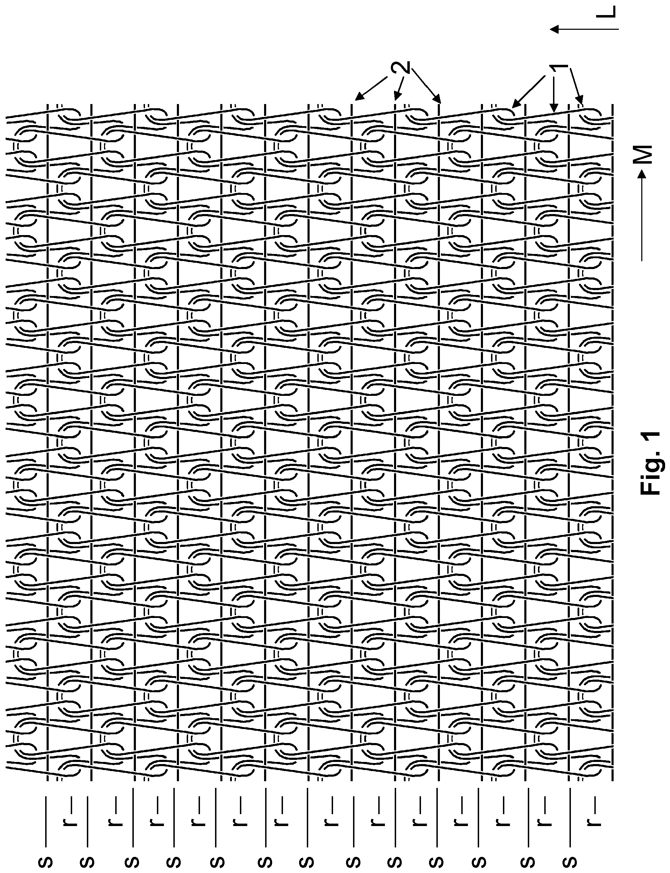

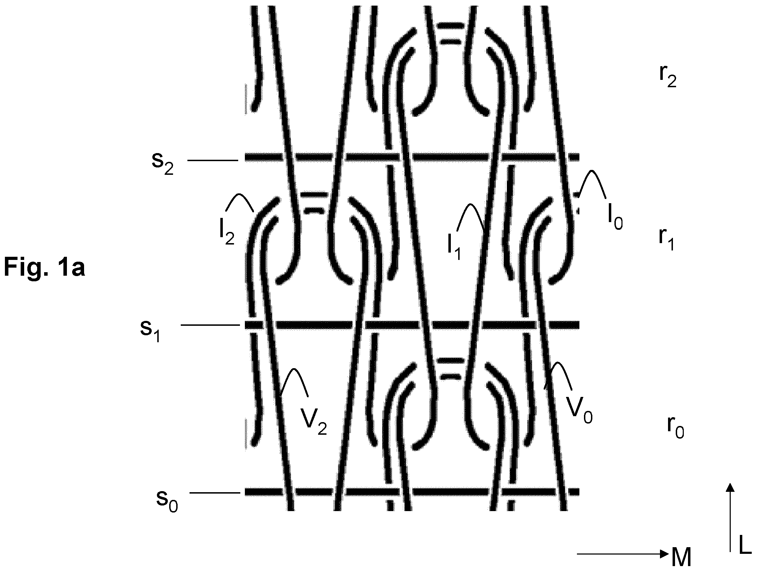

Bei dem nur beispielhaft zur Erläuterung der Erfindung in den Figuren 1 - 3 gezeigten Ausführungsbeispiel eines erfindungsgemäßen Kompressionsgestricks ist das Grundgestrick aus einem (einzigen) Strickfaden 1 gebildet, wobei das Grundgestrick als Rechts-Links-Gestrick ausgebildet ist. Der Strickfaden 1 ist dabei in jeder Maschenreihe r des Grundgestricks abwechselnd als Masche (I) und Fang (V) gestrickt, so dass der Strickfaden 1 zwischen zwei Maschen (I) einer Maschenreihe r des Grundgestricks jeweils einen Fanghenkel bildet.

Zwischen benachbarten Maschenreihen r des Grundgestricks ist jeweils eine Schussfadenreihe s angeordnet, in der jeweils ein Schussfaden 2 eingelegt ist. Der Schussfaden 2 ist dabei in jeder Schussfadenreihe s abwechselnd als Fang (V) und Flottung (-) eingelegt, so dass der Schussfaden 2 zwischen zwei Fanghenkel (V) einer Schussfadenreihe s jeweils flott liegt.

Die abwechselnde Reihenfolge der Maschen (I) und des Fangs (V) von in Längsrichtung L aufeinanderfolgenden Maschenreihen r des Grundgestricks ist dabei jeweils um eine Masche versetzt angeordnet, wie aus den Figuren ersichtlich, d.h. in Längsrichtung L aufeinanderfolgende Maschenreihen r des Grundgestricks sind die Maschen (I) in Maschenstäbchenrichtung M um eine Masche versetzt angeordnet und entsprechend sind auch die zwischen zwei Maschen (I) liegenden Fanghenkel (V) in Maschenstäbchenrichtung M um eine Masche versetzt. Entsprechend ist auch die abwechselnde Reihenfolge von Fang (V) und Flottung (-) des Schussfadens 2 in aufeinanderfolgenden Schussfadenreihen s jeweils um eine Masche versetzt.

Durch die jeweils um eine Masche versetzte Anordnung der Reihenfolge von Masche (I) und Fang (V) in aufeinanderfolgenden Maschenreihen r des Grundgestricks und die abwechselnde Reihenfolge von Fang (V) und Flottung (-), die in aufeinanderfolgenden Schussfadenreihen s ebenfalls jeweils um eine Masche versetzt ist, wird der elastische Schussfaden zwar zunächst in jeder Schussfadenreihe s abwechselnd als Fang (V) und als Flottung (-) eingelegt. Die darauffolgende Masche (I) (in der darauffolgenden Maschenreihe r des Grundgestricks) wirft den elastischen Schussfaden 2 jedoch ab und somit entsteht an dieser Stelle daraus eine Flottung (-) des Schussfadens 2.

Die EP 1 895 036 A1 zeigt ein gattungsgemäßes Kompressionsgestrick für einen medizinischen Kompressionsartikel, welches ein Grundgestrick mit Maschen, Fanghenkeln und Flottungen umfasst, wobei ein Schussfaden in das Grundgestrick eingelegt sein kann, um einen kompressiven Bereich des Gestricks auszubilden.

Aus der US 3,975,929 ist ein rundgestrickter Strumpf bekannt, wobei das Rundgestrick einen Elastan-Faden sowie ein dehnbares, texturiertes Garn enthält, welche alternierend zu Maschen gestrickt und Fanghenkel bildend verstrickt sind, um in einem oberen Bereich des Strumpfs einen vergrößerten Umfang auszubilden.

Die DE 2 047 566 A offenbart ein Verfahren zur Herstellung einfonturiger Kulierware.

Die US 3,287,938 zeigt elastische Gestricke, welche ein 1x1-Versatzmuste von Fanghenkeln und Strickmaschen als Grundgestrick beinhalten.

Wie aus der Detaildarstellung der Figur 1a ersichtlich, welche einen vergrößerten Ausschnitt des Maschenbilds der Figur 1 auf der rechten Warenseite zeigt, liegt der Schussfaden 2 in einer bestimmten Schussfadenreihe s₁ hinter einer ersten Masche I₁ aus der benachbarten Maschenreihe r₁ des Grundgestricks und vor der zu dieser ersten Masche I₁ in Maschenstäbchenrichtung M benachbarten zweiten Masche I₂. Gleichzeitig liegt der Schussfaden 2 hinter dem aus der darunterliegenden Maschenreihe ro hochgezogenen Fanghenkel V₂, so dass der Schussfaden 2 in der bestimmten Schussfadenreihe s₁ zwischen der benachbarten zweiten Masche I₂ und dem hochgezogenen Fanghenkel V₂ durchgreift.

Wie aus Figur 3 ersichtlich, weist das Gestrick einen sowohl in Maschenreihenrichtung M als auch in Längsrichtung L des Gestricks periodisch wiederkehrenden Rapport R auf. Der Rapport R kann sich dabei insbesondere in Maschenreihenrichtung M über vier Maschen und in Längsrichtung L über vier aufeinanderfolgende Reihen (also über zwei Maschenreihen r des Grundgestricks und zwei Schussfadenreihen s) erstrecken.

Wie aus dem Maschenbild der Figur 1 ersichtlich, liegt der Schussfaden dadurch über seine gesamte Länge (die sich in Maschenreihenrichtung M erstreckt) frei und ohne Bindungspunkte in dem Grundgestrick. Der elastische Schussfaden 2 ist deshalb in dem Grundgestrick frei beweglich. Der elastische Schussfaden 2 erzeugt durch seine Elastizität eine Dehnbarkeit des Gestricks in Maschenreihenrichtung M, wodurch beispielsweise bei einem als Rundgestrick ausgebildeten Kompressionsgestrick eine Kompressionswirkung in Umfangsrichtung des schlauchförmigen Rundgestricks erzeugt wird.

Der Strickfaden 1, bei dem es sich auch um einen elastischen Faden handeln kann, trägt bei Verwendung eines elastischen Strickfadens im geringem Umfang ebenfalls zur Dehnbarkeit und damit zur Ausbildung einer Kompressionswirkung des Kompressionsgestricks bei, allerdings in wesentlich geringerem Maße als der frei im Grundgestrick eingelegte elastische Schussfaden 2.

Dadurch, dass der Schussfaden ohne Bindungspunkte frei in dem Grundgestrick eingelegt ist, wird die Elastizität des elastischen Schussfadens 2 bei einer Dehnung des Kompressionsgestricks in Maschenreihenrichtung M nicht behindert. Dadurch kann eine genau definierte Dehnbarkeit, die ausschließlich von der Elastizität des Schussfadens 2 abhängt, verwirklicht werden. Bei der Herstellung von Kompressionsartikeln aus dem erfindungsgemäßen Kompressionsgestrick lassen sich daher genau definierte Kompressionsdrücke erzeugen. Insbesondere bei Kompressionsstrümpfen, die einen von distal nach proximal abnehmenden Kompressionsdruckverlauf aufweisen, ist dadurch eine genaue Einstellung eines medizinisch gewünschten Druckgradienten möglich.

Aus dem Maschenbild von Figur 1 ist weiterhin ersichtlich, dass der Schussfaden 2 über seine gesamte Länge, die sich in Maschenreihenrichtung M erstreckt, innerhalb des Grundgestricks liegt. Der elastische Schussfaden 2 ist damit anders als bei den bekannten Kompressionsgestricken vollständig innenliegend. Dies verbessert die Anziehbarkeit eines aus dem erfindungsgemäßen Kompressionsgestrick hergestellten Kompressionsartikels aufgrund einer verminderten Reibung zwischen der Gestrickinnenseite des Kompressionsartikels und der Haut eines Patienten.

Das Kompressionsgestrick gemäß der Erfindung kann in Kompressionsartikeln auch nur bereichsweise eingesetzt werden und dabei mit anderen Gestrickbindungen kombiniert werden. So kann bspw. ein erfindungsgemäß ausgebildeter Abschnitt mit einem Gestrickabschnitt aus einem herkömmlichen Kompressionsgestrick oder mit Gestrickabschnitten ohne eingelegten Schussfaden nahtlos verstrickt sein.

## Patentansprüche

1. Kompressionsgestrick bestehend aus einem Grundgestrick und einem darin eingelegten elastischen Schussfaden (2), wobei das Grundgestrick aus wenigstens einem Strickfaden (1) gebildet ist und der Schussfaden (2) jeweils in Schussfadenreihen (s) zwischen zwei aufeinanderfolgenden Maschenreihen (r) des Grundgestricks eingelegt ist, **dadurch gekennzeichnet, dass**
- der Strickfaden (1) in jeder Maschenreihe (r) des Grundgestricks abwechselnd als Masche (I) und Fang (V) gestrickt ist, so dass der Strickfaden (1) zwischen zwei Maschen (I) einer Maschenreihe (r) des Grundgestricks jeweils einen Fanghenkel bildet,
- der Schussfaden (2) zwischen benachbarten Maschenreihen (r) des Grundgestricks abwechselnd als Fang (V) und Flottung (-) eingelegt ist, so dass der Schussfaden (2) zwischen zwei Fanghenkel (V) einer Schussfadenreihe (s) jeweils flott liegt,
- wobei die abwechselnde Reihenfolge von Masche (I) und Fang (V) in aufeinanderfolgenden Maschenreihen (r) des Grundgestricks gegenüber der abwechselnden Reihenfolge von Fang (V) und Flottung (-) in aufeinanderfolgenden Schussfadenreihen (s) jeweils um eine Masche versetzt ist.

2. Kompressionsgestrick nach Anspruch 1, **dadurch gekennzeichnet, dass** der Strickfaden (1) elastisch ist.

3. Kompressionsgestrick nach Anspruch 2, **dadurch gekennzeichnet, dass** es sich bei dem elastischen Strickfaden (1) um ein Umwindegarn mit einem elastischen Kernfaden handelt.

4. Kompressionsgestrick nach Anspruch 3, wobei der Strickfaden (1) eine geringere Dicke als der Schussfaden (2) aufweist.

5. Kompressionsgestrick nach einem der voranstehenden Ansprüche, **dadurch gekennzeichnet, dass** es sich bei dem elastischen Schussfaden (2) um ein Umwindegarn mit einem hochelastischen Kernfaden oder um einen Elasthan- oder Gummifaden handelt.

6. Kompressionsgestrick nach einem der voranstehenden Ansprüche, wobei der Schussfaden (2) eine Dicke im Bereich von 200 bis 1500 dtex aufweist.

7. Kompressionsgestrick nach einem der voranstehenden Ansprüche, **dadurch gekennzeichnet, dass** der Schussfaden (2) in dem Grundgestrick ohne Bindungspunkte eingelegt ist.

8. Kompressionsgestrick nach einem der voranstehenden Ansprüche, **dadurch gekennzeichnet, dass** der Schussfaden (2) in dem Kompressionsgestrick vollständig innenliegend ist und nicht an der Oberfläche erscheint.

9. Kompressionsgestrick nach einem der voranstehenden Ansprüche, **dadurch gekennzeichnet, dass** es sich bei dem Grundgestrick um ein rechts-links-Gestrick handelt.

10. Kompressionsgestrick nach einem der voranstehenden Ansprüche, **dadurch gekennzeichnet, dass** es ein Rundgestrick ist.

11. Kompressionsgestrick nach einem der voranstehenden Ansprüche, **dadurch gekennzeichnet, dass** es einen sowohl in Maschenreihenrichtung (M) als auch in Längsrichtung (L) des Gestricks periodisch wiederkehrenden Rapport (R) aufweist.

12. Kompressionsgestrick nach einem der voranstehenden Ansprüche, **dadurch gekennzeichnet, dass** der Schussfaden (2) in einer bestimmten Schussfadenreihe (s₁) hinter einer ersten Masche (I₁) aus der benachbarten Maschenreihe (r₁) des Grundgestricks liegt und vor der zu dieser Masche (I₁) in Maschenstäbchenrichtung (M) benachbarten zweiten Masche (I₂) und gleichzeitig hinter dem aus der darunterliegenden Maschenreihe (r₀) hochgezogenen Fanghenkel (V₂) liegt, so dass der Schussfaden (2) in der bestimmten Schussfadenreihe (s₁) zwischen der benachbarten zweiten Masche (I₂) und dem Fanghenkel (V₂) durchgreift.

13. Kompressionsartikel, insbesondere Kompressionsstrumpf oder -ärmel, hergestellt aus einem Kompressionsgestrick nach einem der voranstehenden Ansprüche.

14. Verfahren zur Herstellung eines Kompressionsgestricks aus einem Grundgestrick und einem darin eingelegten elastischen Schussfaden (2), wobei das Grundgestrick aus wenigstens einem Strickfaden (1) gestrickt und der Schussfaden (2) jeweils in Schussfadenreihen (s) zwischen zwei aufeinanderfolgenden Maschenreihen (r) des Grundgestricks eingelegt wird, **dadurch gekennzeichnet, dass**
- der Strickfaden (1) in jeder Maschenreihe (r) des Grundgestricks abwechselnd als Masche (I) und Fang (V) gestrickt wird, so dass der Strickfaden (1) zwischen zwei Maschen (I) einer Maschenreihe (r) des Grundgestricks jeweils einen Fanghenkel bildet,
- der Schussfaden (2) zwischen benachbarten Maschenreihen (r) des Grundgestricks abwechselnd als Fang (V) und Flottung (-) eingelegt wird, so dass der Schussfaden (2) zwischen zwei Fanghenkel (V) einer Schussfadenreihe (s) jeweils flott liegt,
- wobei die abwechselnde Reihenfolge von Masche (I) und Fang (V) in aufeinanderfolgenden Maschenreihen (r) des Grundgestricks gegenüber der abwechselnden Reihenfolge von Fang (V) und Flottung (-) in aufeinanderfolgenden Schussfadenreihen (s) jeweils um eine Masche versetzt ist.

15. Verfahren nach Anspruch 14, **dadurch gekennzeichnet, dass** die Fanghenkel des elastischen Schussfadens (2) durch die Einlegung des elastischen Schussfadens zwischen zwei aufeinanderfolgenden Maschenreihen (r₀, r₁) des Grundgestricks in einer Masche (I) der darauf folgenden Maschenreihe (r₁) des Grundgestricks abgeworfen werden und der Schußfaden (2) somit an diesen Stellen flott liegt.

## Claims

1. Compression knitted fabric consisting of a base knitted fabric and an elastic weft thread (2) inserted therein, wherein the base knitted fabric is formed from at least one knitting thread (1) and the weft thread (2) is inserted respectively in weft thread rows (s) between two consecutive stitch rows (r) of the base knitted fabric, **characterised in that**
- the knitting thread (1) in each stitch row (r) of the base knitted fabric is knitted alternately as stitch (I) and tuck (V), so that the knitting thread (1) forms respectively a tuck stitch between two stitches (I) of a stitch row (r) of the base knitted fabric,
- the weft thread (2) is inserted between adjacent stitch rows (r) of the base knitted fabric alternately as tuck (V) and float (-), so that the weft thread (2) respectively floats between two tuck stitches (V) of a weft thread row (s),
- wherein the alternate sequence of stitch (I) and tuck (V) in consecutive stitch rows (r) of the base knitted fabric is offset respectively by one stitch with respect to the alternate sequence of tuck (V) and float (-) in consecutive weft thread rows (s).

2. Compression knitted fabric according to claim 1, **characterised in that** the knitting thread (1) is elastic.

3. Compression knitted fabric according to claim 2, **characterised in that** the elastic knitting thread (1) is a winding yarn with an elastic core thread.

4. Compression knitted fabric according to claim 3, wherein the knitting thread (1) has a lower thickness than the weft thread (2).

5. Compression knitted fabric according to one of the preceding claims, **characterised in that** the elastic weft thread (2) is a winding yarn with a highly elastic core thread or an elastane thread or rubber thread.

6. Compression knitted fabric according to one of the preceding claims, wherein the weft thread (2) has a thickness in the range from 200 to 1,500 dtex.

7. Compression knitted fabric according to one of the preceding claims, **characterised in that** the weft thread (2) is inserted in the base knitted fabric without binding points.

8. Compression knitted fabric according to one of the preceding claims, **characterised in that** the weft thread (2) lies fully on the inside in the compression knitted fabric and does not appear on the surface.

9. Compression knitted fabric according to one of the preceding claims, **characterised in that** the base knitted fabric is a right-left knitted fabric.

10. Compression knitted fabric according to one of the preceding claims, **characterised in that** it is a circular knitted fabric.

11. Compression knitted fabric according to one of the preceding claims, **characterised in that** it has a periodically recurring repeat (R) both in the stitch row direction (M) and in the longitudinal direction (L) of the knitted fabric.

12. Compression knitted fabric according to one of the preceding claims, **characterised in that** the weft thread (2) in a certain weft thread row (s₁) lies behind a first stitch (I₁) from the adjacent stitch row (r₁) of the base knitted fabric and lies in front of the second stitch (I₂) adjacent to this stitch (I₁) in the stitch wale direction (M) and at the same time behind the tuck stitch (V₂) raised from the underlying stitch row (r₀), so that the weft thread (2) in the certain weft thread row (s₁) passes between the adjacent second stitch (I₂) and the tuck stitch (V₂).

13. Compression article, in particular compression stocking or compression sleeve, produced from a compression knitted fabric according to one of the preceding claims.

14. Method for producing a compression knitted fabric from a base knitted fabric and an elastic weft thread (2) inserted therein, wherein the base knitted fabric is knitted from at least one knitting thread (1) and the weft thread (2) is inserted respectively in weft thread rows (s) between two consecutive stitch rows (r) of the base knitted fabric, **characterised in that**
- the knitting thread (1) in each stitch row (r) of the base knitted fabric is knitted alternately as stitch (I) and tuck (V), so that the knitting thread (1) forms respectively a tuck stitch between two stitches (I) of a stitch row (r) of the base knitted fabric,
- the weft thread (2) is inserted between adjacent stitch rows (r) of the base knitted fabric alternately as tuck (V) and float (-), so that the weft thread (2) respectively floats between two tuck stitches (V) of a weft thread row (s),
- wherein the alternate sequence of stitch (I) and tuck (V) in consecutive stitch rows (r) of the base knitted fabric is offset respectively by one stitch with respect to the alternate sequence of tuck (V) and float (-) in consecutive weft thread rows (s).

15. Method according to claim 14, **characterised in that** the tuck stitches of the elastic weft thread (2) are discarded by insertion of the elastic weft thread between two consecutive stitch rows (r₀, r₁) of the base knitted fabric in a stitch (I) of the subsequent stitch row (r₁) of the base knitted fabric and the weft thread (2) thus floats at these points.

## Revendications

1. Tricot de compression se composant d'un tricot de base et d'un fil de trame élastique (2) qui y est introduit, dans lequel le tricot de base est formé d'au moins un fil de tricot (1) et le fil de trame (2) est introduit respectivement en rangées de fil de trame (s) entre deux rangées de mailles (r) successives du tricot de base, **caractérisé en ce que**
- le fil de tricot (1) est tricoté dans chaque rangée de mailles (r) du tricot de base en alternance en tant que maille (I) et charge (V), de sorte que le fil de tricot (1) entre deux mailles (I) d'une rangée de mailles (r) du tricot de base forme respectivement une boucle de cueillage,
- le fil de trame (2) entre des rangées de mailles (r) adjacentes du tricot de base est introduit en alternance en tant que charge (V) et flottage (-), de sorte que le fil de trame (2) est respectivement flottant entre deux boucles de cueillage (V) d'une rangée de fil de trame (s),
- dans lequel l'ordre alterné de maille (I) et de charge (V) dans des rangées de mailles (r) successives du tricot de base est décalé respectivement d'une maille par rapport à l'ordre alterné de charge (V) et de flottage (-) dans des rangées de fil de trame (s) successives.

2. Tricot de compression selon la revendication 1, **caractérisé en ce que** le fil de tricot (1) est élastique.

3. Tricot de compression selon la revendication 2, **caractérisé en ce que** le fil de tricot élastique (1) est un fil de guipage avec un fil d'âme élastique.

4. Tricot de compression selon la revendication 3, dans lequel le fil de tricot (1) présente une épaisseur inférieure à celle du fil de trame (2).

5. Tricot de compression selon l'une quelconque des revendications précédentes, **caractérisé en ce que** le fil de trame élastique (2) est un fil de guipage avec un fil d'âme très élastique ou un fil d'élasthanne ou de caoutchouc.

6. Tricot de compression selon l'une quelconque des revendications précédentes, dans lequel le fil de trame (2) présente une épaisseur de l'ordre de 200 à 1500 dtex.

7. Tricot de compression selon l'une quelconque des revendications précédentes, **caractérisé en ce que** le fil de trame (2) est introduit dans le tricot de base sans points de liaison.

8. Tricot de compression selon l'une quelconque des revendications précédentes, **caractérisé en ce que** le fil de trame (2) est complètement à l'intérieur dans le tricot de base et n'apparaît pas à la surface.

9. Tricot de compression selon l'une quelconque des revendications précédentes, **caractérisé en ce que** le tricot de base est un tricot droite-gauche.

10. Tricot de compression selon l'une quelconque des revendications précédentes, **caractérisé en ce qu'**il s'agit d'un tricot rond.

11. Tricot de compression selon l'une quelconque des revendications précédentes, **caractérisé en ce qu'**il présente un rapport récurrent périodiquement (R) aussi bien dans la direction de la rangée de mailles (M) que dans la direction longitudinale (L) du tricot.

12. Tricot de compression selon l'une quelconque des revendications précédentes, **caractérisé en ce que** le fil de trame (2) se trouve dans une rangée de fil de trame (s₁) déterminée derrière une première maille (I₁) de la rangée de mailles adjacente (r₁) du tricot de base et devant la deuxième maille (I₂) adjacente à cette maille (I₁) dans la direction des colonnes de mailles (M) et en même temps derrière la boucle de cueillage (V₂) tirée de la rangée de mailles sous-jacente (r₀), de sorte que le fil de trame (2) traverse dans la rangée de fil de trame (s₁) déterminée entre la deuxième maille (I₂) adjacente et la boucle de cueillage (V₂).

13. Article de contention, en particulier collant ou manchon de contention fabriqué en un tricot de compression selon l'une quelconque des revendications précédentes.

14. Procédé de fabrication d'un tricot de compression d'un tricot de base et d'un fil de trame élastique (2) qui y est introduit, dans lequel le tricot de base est tricoté à partir d'au moins un fil de tricot (1) et le fil de trame (2) est introduit respectivement en rangées de fil de trame (s) entre deux rangées de mailles (r) successives du tricot de base, **caractérisé en ce que**
- le fil de tricot (1) est tricoté dans chaque rangée de mailles (r) du tricot de base en alternance en tant que maille (I) et charge (V), de sorte que le fil de tricot (1) entre deux mailles (I) d'une rangée de mailles (r) du tricot de base forme respectivement une boucle de cueillage,
- le fil de trame (2) entre des rangées de mailles (r) adjacentes du tricot de base est introduit en alternance en tant que charge (V) et flottage (-), de sorte que le fil de trame (2) est respectivement flottant entre deux boucles de cueillage (V) d'une rangée de fil de trame (s),
- dans lequel l'ordre alterné de maille (I) et de charge (V) dans des rangées de mailles (r) successives du tricot de base est décalé respectivement d'une maille par rapport à l'ordre alterné de charge (V) et de flottage (-) dans des rangées de fil de trame (s) successives.

15. Procédé selon la revendication 14, **caractérisé en ce que** les boucles de cueillage du fil de trame élastique (2) sont jetées par l'introduction du fil de trame élastique entre deux rangées de mailles (r₀, r₁) successives du tricot de base dans une maille (I) de la rangée de mailles (r₁) successive du tricot de base et le fil de trame (2) est ainsi flottant à ces endroits.
